# EUROPEAN PATENT APPLICATION

(11) **EP 3 815 682 A1**
(43) Date of publication of application: **05.05.2021**
(21) Application number: 20204016.8
(22) Date of filing: 27.10.2020
(51) Int. Cl.: A61K 31/121, A61P 25/16, A61P 25/28, A61P 27/06

(54) **A PERK KINASE INHIBITOR FOR TREATING DISEASES ASSOCIATED WITH ENDOPLASMIC RETICULUM STRESS**

(30) Priority: 28.10.2019 PL 43161319
(71) Applicant: Uniwersytet Medyczny w Lodzi, 90-419 Lodz (PL)
(72) Inventor: Majsterek, Ireneusz, 92-503 Lódz (PL); Rozpedek, Wioletta, 90-644 Lódz (PL); Walczak, Anna, 90-252 Lódz (PL); Kabzinski, Jacek, 91-464 Lódz (PL)
(74) Representative: Godlewski, Piotr

(57) **Abstract**

The object of the present invention is a compound for use as a medicament, a compound for use as the PERK kinase inhibitor for preventing and treating diseases associated with endoplasmic reticulum stress. Preferably, those diseases are selected from a group including glaucoma, Alzheimer's disease, Parkinson's disease.

## Description

The object of the present invention is a compound for use as a medicament, a PERK kinase inhibitor and its use for preventing and treating neurodegenerative diseases associated with endoplasmic reticulum stress.

Neurodegenerative diseases have been known in medicine for a long time and remain a focus of attention of many researchers, as there are still no satisfactory treating methods available. One example could be Alzheimer's disease, Parkinson's disease or glaucoma that, due to being asymptomatic in its first phase and having progressive and irreversible nature is known as an insidious "eyesight thief'.

The 2015 report of Alzheimer's Association shows that in the United States alone, over 2 million people are affected by Alzheimer's disease. Similarly to Alzheimer's disease, glaucoma develops later in life and is currently the leading cause of vision loss in an ageing society. It is estimated that approximately 70 million people worldwide suffer from glaucoma, as reported by studies conducted from 1990 to 2020. Glaucoma was classified as a disease of neurodegenerative origin and it is one of the main causes of the irreversible vision loss in highly developed countries. Regardless of developments in medicine, the exact cause of its origin is not known. Also, fully effective treating methods are still not available. Based on the studies conducted so far, it is believed that one of the pathomechanisms involved in neurodegeneration in the ageing process could be a UPR pathway and therefore, specific PERK kinase inhibitors may have potential uses as an active substance in the process of formulating new medicaments (Alzheimer's Association. 2015 Alzheimer's Disease Facts and Figures. Alzheimer's & Dementia 2015;11(3)332+; Flaxman SR. Global causes of blindness and distance vision impairment 1990-2020: a systematic review and meta-analysis The Lancet 2017; (17) 30393-5).

The accumulation of abnormally synthesized proteins within the endoplasmic reticulum (ER) activates a cell response mechanism for incorrectly folded proteins (unfolded protein response - UPR) that includes a cascade of events leading to translation silencing, transactivation of ER caretaker gene promoters and activation of protein degradation systems. This subsequently results in inhibition of translation of most proteins as a result of phosphorylation of an eukaryotic initiation factor 2, eIF2α, by the active PERK kinase (protein kinase RNA-like endoplasmic reticulum kinase - PERK). Apart from the global inhibition of protein synthesis by eIF2α phosphorylation, another effect of the PERK-dependent response is the activation of ATF4 transcription factor.

Chronic ER stress due to ATF4 activation contributes to the preferential translation of factors that take part in apoptotic cell death, including CHOP protein (CCAATenhancer-binding protein homologous protein).

The necessity to find new and effective substances of potential use in neurodegenerative diseases was the incentive to test compounds from the group of specific, small molecule inhibitors of transmembrane endoplasmic reticulum kinase, PERK

The first small molecule inhibitor tested in neurodegenerative disease models was the one synthesized by GlaxoSmithKline, GSK2606414, characterized in high selectivity for PERK kinase. Studies conducted in Skirball Institute of Biomolecular Medicine, New York University School of Medicine showed that in a mouse model of prion disease, inhibition of synthesis of most proteins in cells was caused by excessive activation of PERK kinase in intracellular ER stress conditions (Jeffrey M. Axten, Stuart P. Romeril, Arthur Shu, Jeffrey Ralph, Jesuś R. Medina, Yanhong Feng, William Hoi Hong Li, Seth W. Grant, Dirk A. Heerding, Elisabeth Minthorn, Thomas Mencken, Nathan Gaul, Aaron Goetz, Thomas Stanley, Annie M. Hassell, Robert T. Gampe, Charity Atkins, and Rakesh Kumar Discovery of GSK2656157: An Optimized PERK Inhibitor Selected for Preclinical Development ACS Med. Chem. Lett. 2013, 4, 964-968). In mice administered with a GSK2606414 inhibitor, a temporary restoration of protein synthesis in cells was observed. Furthermore, the level of phosphorylated forms of key proteins in the UPR pathway, such as PERK and eIF2α, was substantially lower than in mice that were not administered the inhibitor. However, GSK2606414 was excluded from further studies on the future therapeutic strategy, because analyses, also conducted in Skirball Institute of Biomolecular Medicine, New York University School of Medicine, showed that GSK2606414 has a cytotoxic effect on pancreatic cells in mice. Animals that were administered GSK2606414 showed a significant weight loss and hyperglycemia (Heather P. Harding, Huiqing Zeng, Yuhong Zhang, Rivka Jungries, Peter Chung, Heidi Plesken, David D. Sabatini, and David Ron. Diabetes Mellitus and Exocrine Pancreatic Dysfunction in Perk/Mice Reveals a Role for Translational Control in Secretory Cell Survival. Molecular Cell 2001, 7, 1153-1163).

A second generation PERK inhibitor synthesized by GlaxoSmithKline is GSK2656157 and is characterized by better pharmacokinetics and selectivity for PERK kinase compared to GSK2606414. Research experiments conducted by Charity Atkins et al. showed that GSK2656157 causes an effective inhibition of PERK kinase and eIF2α protein phosphorylation, but similarly to GSK2606414, it was not included in clinical trials because of its non-selective activity also on pancreatic cells (Charity Atkins, Qi Liu, Elisabeth Minthorn, Shu-Yun Zhang, David J. Figueroa, Katherine Moss, Thomas B. Stanley, Brent Sanders, Aaron Goetz, Nathan Gaul, Anthony E. Choudhry, Hasan Alsaid, Beat M. Jucker, Jeffrey M. Axten, and Rakesh Kumar. Characterization of a Novel PERK Kinase Inhibitor with Antitumor and Antiangiogenic Activity. Cancer Res. 2013 73(6): 1993-2012).

Another compound tested in neurodegenerative diseases as the UPR pathway inhibitor dependent on PERK kinase was Salubrinal, selected by Michaela Boyce et al. for further studies based on virtual screening of a database containing approx. 19,000 compounds that inhibit apoptosis caused by ER stress in PC12 cells (Michael Boyce, Kevin F. Bryant, C' eline Jousse, Kai Long, Heather P. Harding, Donalyn Scheuner, Randal J. Kaufman, Dawei Ma, Donald M. Coen, David Ron, Junying Yuan. A Selective Inhibitor of eIF2a Dephosphorylation Protects Cells from ER Stress. Science 2005. 307, 935).

Research experiments conducted by Julie A. Moreno et al. showed that in a mice model of prion disease, Salubrinal did not have the anticipated effect, as it caused an increase in eIF2α phosphorylation. Animals that received Salubrinal died quicker than animals that did not receive Salubrinal (Julie A. Moreno, Helois Radford, Diego Peretti, Joern R. Steinert, Nicholas Verity, Maria Guerra Martin, Mark Halliday, Jason Morgan, David Dinsdale, Catherine A. Ortori, David A. Barrett, Pavel Tsaytler, Anne Bertolotti, Anne E. Willis, Martin Bushell, and Giovanna R. Mallucci. Sustained translational repression by eIF2α-P mediates prion neurodegeneration Nature 2012. 485(7399): 507-511; Julie A. Moreno, Mark Halliday, Colin Molloy, Helois Radford, Nicholas Verity, Jeffrey M. Axten, Catharine A. Ortori, Anne E. Willis, Peter M. Fischer, David A. Barrett, Giovanna R. Mallucci. Oral Treatment Targeting the Unfolded Protein Response Prevents Neurodegeneration and Clinical Disease in Prion-Infected Mice. Science Translational Medicine 2013, 5 (206): 1-10).

Another compound selected at the University of California, San Francisco by Carmela Sidrauski et al. that affected the UPR pathway in cancer disease models was ISRIB, characterized in good permeability of the blood-brain barrier (Carmela Sidrauski, Diego Acosta-Alvear, Arkady Khoutorsky, Punitha Vedantham, Brian R Hearn, Han Li, Karine Gamache, Ciara M Gallagher, Kenny K-H Ang, Chris Wilson, Voytek Okreglak, Avi Ashkenazi, Byron Hann, Karim Nader, Michelle R Arkin, Adam R Renslo, Nahum Sonenberg, Peter Walter. Pharmacological brake-release of mRNA translation enhances cognitive memory. Cell biology | Neuroscience 2013;2:1-22).

Articles published by Mark Halliday and Heather L. Smith et al. show that despite its neuroprotective effect and low cytotoxicity, ISRIB was characterized by low solubility, and therefore it cannot represent a UPR pathway inhibitor effective in the treatment of neurodegenerative diseases (Mark Halliday, Helois Radford, Karlijn A. M. Zents, Collin Molloy, Julie A. Moreno, Nicholas C. Verity, Ewan Smith, Catharine A. Ortori, David A. Barrett, Martin Bushell and Giovanna R. Mallucci. Repurposed drugs targeting eIF2a-P-mediated translational repression prevent neurodegeneration in mice BRAIN 2017: 140; 1768-1783, Heather L. Smith and Giovanna R. Mallucci. The unfolded protein response: mechanisms and therapy of neurodegeneration. BRAIN 2016: 139; 2113-2121).

Examples above show that compounds identified so far with confirmed PERK inhibitor properties cannot be used in therapy because either of their cytotoxicity, or low solubility. Therefore, there is a need to find an effective PERK inhibitor with low and/or minimal cytotoxicity that could be useful in preventing and/or treating neurodegenerative diseases based on a PERK inhibition mechanism, especially glaucoma.

Known small molecule compounds are deposited in several bases and libraries, such as the LDDN base (Laboratory for Drug Discovery in Neurodegeneration) or the American library of small molecule compounds of NCI (National Cancer Institute). One of the known small molecule compounds mentioned above is the compound no 42233 deposited in the NCI library under the systematic name: 3-acetyl-6-methyl-2,4-heptanedione.

Application US2011230564 A1 of prior art discloses that 3-acetyl-6-methyl-2,4-heptanedione has adenine nucleotide translocase 4 - ANT4 inhibitor properties and can be used in treatment a subject suffering from or susceptible to a disorder or disease associated with cell proliferation, such as cancers. Also, US2011230564 A1 discloses that 3-acetyl-6-methyl-2,4-heptanedione may be useful in aiding contraceptive methods by decreasing or eliminating male meiotic spermatocytes (without damaging other cell types) in a subject.

The object of the invention is to provide an effective compound that may be used for preventing and/or treating neurodegenerative disorders based on the PERK inhibition mechanism, especially glaucoma, as well as the use of such compound to create a medicinal product and a medicament for preventing and/or treating such diseases.
The object of the invention is the compound of formula 1: 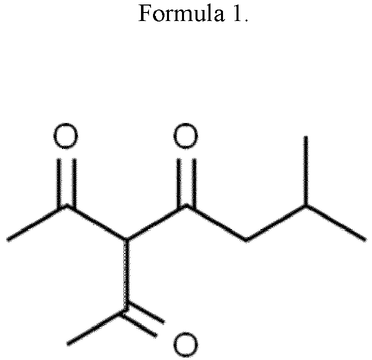 for use as the PERK kinase inhibitor.

Preferably, the compound of formula 1 is in a salt form.

Preferably, the compound of formula 1 is for use in preventing and/or treating diseases associated with endoplasmic reticulum stress.

Preferably, diseases associated with endoplasmic reticulum stress are selected from a group comprising glaucoma, Alzheimer's disease, Parkinson's disease.

Another object of the invention is the compound of formula 1: 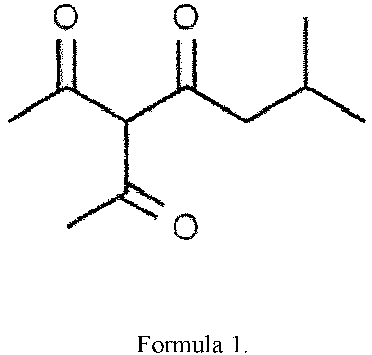 for use in preventing and/or treating diseases associated with endoplasmic reticulum stress.

Preferably, the compound of formula 1 is in a salt form.

Preferably, diseases associated with endoplasmic reticulum stress are selected from a group comprising glaucoma, Alzheimer's disease, Parkinson's disease.
Wherein according to the invention by PERK kinase inhibitor is meant a compound inhibiting the function of protein kinase RNA-like endoplasmic reticulum kinase.
The present invention provides a number of the following benefits:
- The claimed invention is a selective and effective PARK inhibitor;
- The claimed invention is not cytotoxic;
- The synthesis of this compound is not complicated and does not require expensive equipment, which is highly beneficial for preparing the compound on a commercial scale.

The synthesis and study results for compound (I) were shown in the drawing, wherein Figure 1 shows the synthesis of 3-acetyl-6-methyl-2,4-heptanedione, wherein (A) shows the preparation of 6-methylheptane-2,4-dione and (B) shows the conversion of 6-methylheptane-2,4-dione to 3-acetyl-6-methyl-2,4-heptanedione; Figure 2 shows molecular docking results for 3-acetyl-6-methyl-2,4-heptanedione; Figure 3 shows the analysis of the phosphorylation level of PERK and eIF2α in vitro in the presence of 3-acetyl-6-methyl-2,4-heptanedione, designated as 42233; Figure 4 shows the analysis of the activity of 3-acetyl-6-methyl-2,4-heptanedione, designated as 42233, in rat astrocytes DI TNC1; Figure 5 shows the analysis of the eif2a phosphorylation level in response to the 3-acetyl-6-methyl-2,4-heptanedione activity, designated as 42233, in human embryonic kidney cells; Figure 6 shows the analysis of the eif2a phosphorylation level in response to 3-acetyl-6-methyl-2,4-heptanedione, designated as 42233, in mice fibroblasts; Figure 7 shows the analysis of the PERK phosphorylation level in response to 3-acetyl-6-methyl-2,4-heptanedione, designated as 42233, in mice neuronal cells CATH.a; Figure 8 shows the analysis of the CHOP protein level and eIF2α phosphorylation in response to 3-acetyl-6-methyl-2,4-heptanedione, designated as 42233, in rat astrocytes DI TNC1; Figure 9 shows the assessment of 3-acetyl-6-methyl-2,4-heptanedione cytotoxicity in rat astrocytes DI TNC1 incubated for 3, 16, 24 and 48 hours with cytotoxicity assayed by an XTT test; Figure 10 shows the assessment of the 3-acetyl-6-methyl-2,4-heptanedione influence on apoptosis level in rat astrocytes DI TNC1 using flow cytometry; Figure 11 shows the analysis of cell cycle in response to 3-acetyl-6-methyl-2,4-heptanedione in DI TNC1 cells after incubating for 24 hours.

The invention is shown in detail in the following embodiments, wherein all experimental tests and procedures described above were conducted using commercially available testing sets, reagents and apparatus, according to manufacturers' instructions of those testing sets, reagents and apparatus, unless indicated otherwise. All testing parameters were measured with standard and widely known methods used in the field of the present invention.

### Example 1

### Synthesis of 3-acetyl-6-methyl-2,4-heptanedione (compound 42233)

3-acetyl-6-methyl-2,4-heptanedione (compound 42233) can be prepared using well-known methods.

In this embodiment, magnesium shavings (26.7 g), absolute ethanol (135 ml) and carbon tetrachloride (5 ml) were placed in a 2 litre round-bottom flask and gently heated until the release of hydrogen stopped. Dry benzene (200 ml) was added to the stirred mixture and heating was continued until the release of gas stopped altogether (approximately 4 hours). Diethyl ether (500 ml) was added to a cooled mixture and then tert-butyl acetoacetic acid (161 g) was added at a rate that would ensure that mild reflux is maintained. The reaction mixture was heated at a boiling point for additional 30 minutes. The mixture was cooled to room temperature and treated with a slow addition of isovaleryl chloride (120.5 g).

After the reaction was completed, the mixture was heated until boiling point under a condenser for 1 hour and left overnight. The solution was cooled in an ice bath and treated with 5M of sulphuric acid (200 ml) and the amount of water sufficient to dissolve the residue. The organic layer was separated and the aqueous phase was further extracted with ether (3x50 ml). Combined organic extracts were washed with saturated with sodium chloride solution (3x200 ml) and dried under sodium sulphate. Solvents were removed under decreased pressure and the residue oil was treated with p-toluenesulphonic acid (1.2 g) and heated at 150°C until the release of carbon dioxide stopped (about 2 hours).

The product was purified by distillation and collected as a pale yellow oil (78.9 g; 56% yield), boiling temperature 178-188°C; IR (no additions): v 1615 cm⁻¹ (s, sh, C = O).

6-methylheptane-2,4-dione thus obtained was treated with ethenone to give 3-acetyl-6-methyl-2,4-heptanedione.

Anhydride potassium carbonate (95 g) was added to a mixture of 6-methyl-2,4-heptanedione 25c (30.83 g) and ethenone (47.44 g) in acetone of reagent grade purity (140 ml). The mixture was vigorously mixed under a condenser at 80°C overnight. The cooled mixture was filtered and solid potassium carbonate was liberally washed with acetone. The solvent was removed under vacuum and the residual oil was purified by distillation to give the desired diketone as a pale-yellow oil (25.59 g; 76%), boiling temperature 150-152°C.

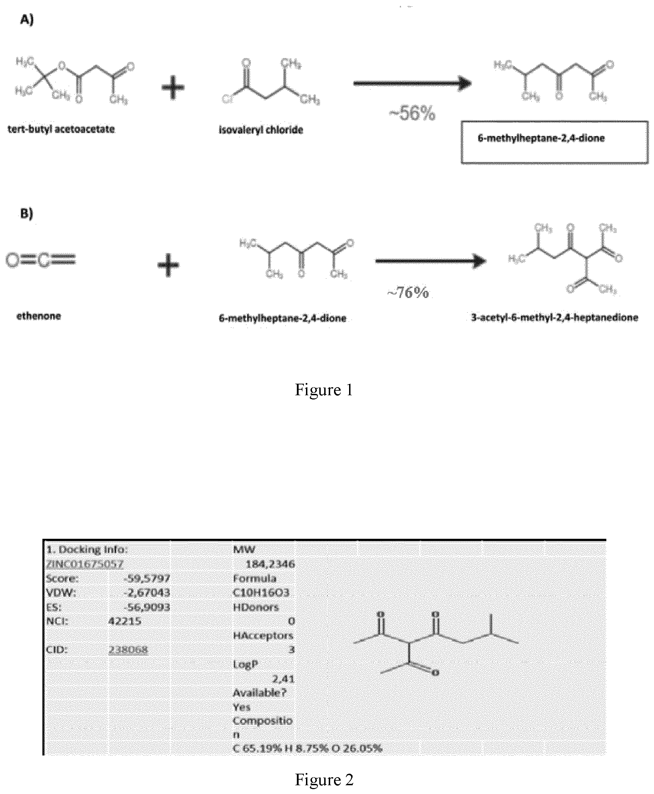

The synthesis of 3-acetyl-6-methyl-2,4-heptanedione according to the embodiment was illustrated in Figure 1 and basic data regarding this compound were shown in Table 1.

**Table 1: basic data regarding 3-acetyl-6-methyl-2,4-heptanedione**

| Product name | **3-acetyl-6-methylheptane-2,4-dione** |
|---|---|
| Molecular formula | C₁₀H₁₆O₃ |
| Molecular weight | 184.2322 |
| InChI | InChI = 1 / C10H16O3 / c1-6 (2) 5-9 (13) 10 (7 (3) 11) 8 (4) 12 / h6,10H, 5H2,1-4H3 |
| CAS registry number | 6303-07-7 |
| Molecular structure | 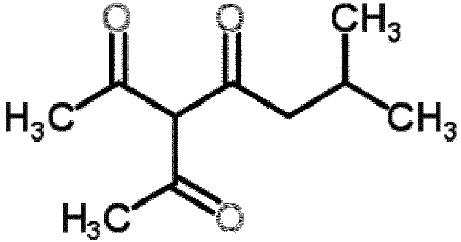 |

### Example 2

### Initial determination of the mechanism of action using molecular docking

To initially confirm the mechanism of action of 3-acetyl-6-methyl-2,4-heptanedione (compound 42233) of formula I: 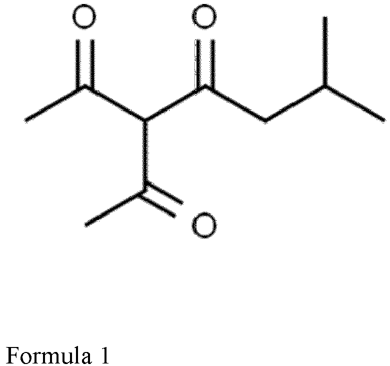 a virtual screening that included computer docking was made.

The strategy of searching for an effective PERK inhibitor was compatible with the methodology of a computer-aided drug design (CADD). Studies included virtual screening (VS), which is a computer technique for automatically browsing and assessing potential biological activity (ranking) in large databases of chemical compounds using chemo-computing, bio-computing tools and molecular modelling.

In this embodiment, the search and analysis for potential small molecule compounds of unknown properties was made based on known and/or predicted receptor structure (structure-based virtual screening, SBVS) during computer docking of a base containing >200 000 compounds included in the library of small molecule compounds library of National Cancer Insititute (NCI) and Laboratory for Drug Discovery in Neurodegeneration (LDDN). For this, the available PERK kinase structure (Homo sapiens, 4YJZS) was used from Protein Data Bank (RCSB PDB) database: http://www.rcsb.org/structure/4YZS.

Then inhibitors were designed based on the structure of known ligands (ligand-based design) and the analysis of spacial features of fields - 3D QSAR (3D quantitative structure activity relationship). For this purpose, the Open3d qsar software was used. This allowed the development of 80,000 small molecule compounds with potential inhibitory properties against PERK kinase.

Then, the structure of 80,000 derivatives of PERK kinase inhibitor was analysed. The object of this part of the step is to select a compound with high inhibitory properties for PERK kinase, characterized in the lowest Docking Score factor. Furthermore, the physicochemical properties of a potential medicinal substance were assessed, which determine the rate of dissolution and the rate of permeation through biological membranes, and as a result, the probability of reaching a concentration considered as therapeutic at the site of action.

Modifications were made using the theory of bioisosterism. Bioisosteres are substituents or groups showing similar physical and generally biological properties. Bioisosterism can be used to modify the main structure, which proved to be an effective way to reduce toxicity or changes in the activity of the main structure. This theory was presented in 1925 by Grimm, who formulated the hydride displacement law. All structure modifications were made according to rules proposed by Lipinski et al. that served to create the so-called rule 5. According to this rule, a potential therapeutic compound should be characterized by having molar mass <500MW, a partition coefficient of two non-combining phases (n-octanol/water) logP < 5, the number of hydrogen bond donors (the number of heteroatoms bound with at least one H atom) HBD<5, and the number of hydrogen bond acceptors (the aggregate number of heteroatoms) HBA <10.

The obtained docking scores (Figure 2) for the analysed compound showed that it has good ligand-receptor binding properties.

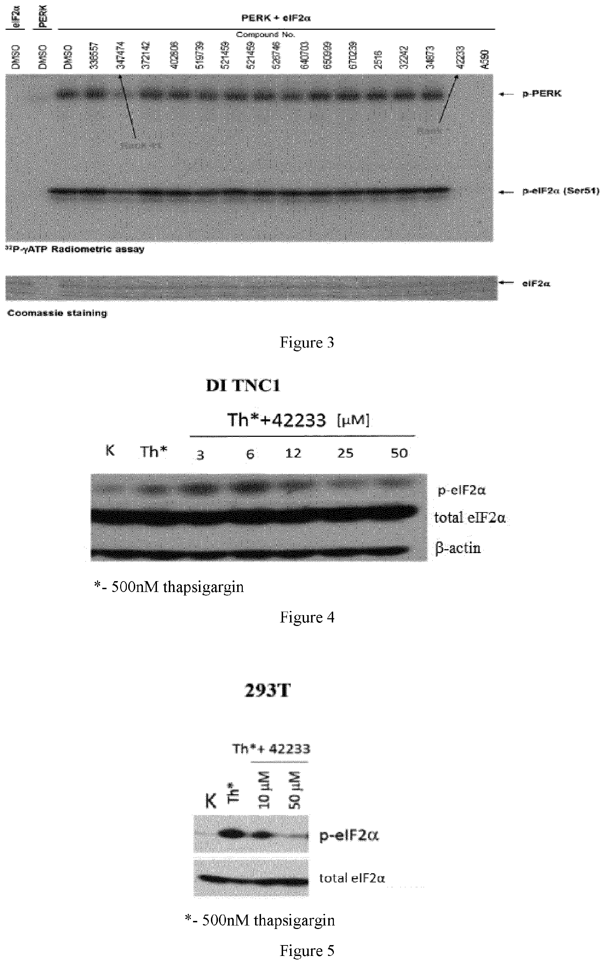

The object of further analysis was to prove the activity of the tested compound in a kinase assay in vitro using a P32 isotope (Figure 3).

During the further screening tests based on (a) a radiometric kinase test, and (b) a TRF-FRET test, a compound that had the strongest inhibitory properties of PERK kinase activity and its main substrate, eIF2α, was selected and further analysed.

### (a) Radiometric Kinase Reaction

In order to characterize enzyme kinetics and establish optimal test conditions, a radiometric assay was used. As a result of reaction optimization, the following content of the reaction mixture was established: 20mM HEPES (pH 7.2), 10 mM MgCh, 150 mM NaCl and 0.01% Tween 20 and 1 µM ATP/1 µCi [γ-33P] ATP, 1 µM eIF2α with 8 nM PERK.

The reaction was carried out for 90 minutes at room temperature and quenched by 75 mM of phosphoric acid. Reaction mixtures were transferred to PH Multiscreen filtration plates (Merck) and then vigorously washed. Filtration plates were dried and the formulation was quantitated by using a scintillation counter. Background (negative control) for the product formulation was tested in the same reaction mixture without the presence of a PERK protein. The optimal concentration of the enzyme was estimated for a value, for which the phospho-eIF2a formation reaction progress graph was linear for at least 60 minutes.

### (b) TR-FRET and HTS (Time-Resolved Fluorescence Resonance Energy Transfer Base Assay and HTS)

To screen a small molecule compounds library, a TR-FRET assay (Z '> 0.7% coefficient of variation [CV] of the positive and negative control << 10% and signal/background level [S/B] of 2.45) was made on 384-well plates. This test enables the measure of direct eIF2α phosphorylation - a specific substrate for PERK kinase using a phospho-specific antibody conjugated with a fluorescent donor of europium cryptate (Eu3+). TR-FRET kinase assay was conducted in a buffer containing 20 mM HEPES (pH 7.2), 10 mM MgCh, 150 mM NaCl and 0.01% Tween 20. All reagents of kinase reaction, such as PERK, eIF2α and ATP, were prepared in the same buffer. PERK enzyme (in its final concentration indicated using a radiometric kinase assay) was pre-incubated for 30 minutes at room temperature with potential inhibitors that were tested (final concentration 10 µM) in 2 µl. After the initial incubation, 1 µl of the eIF2α/ATP mixture was added to initiate kinase reaction (final concentration 1 µM of eIF2α substrate and 1 µM ATP) and was incubated for 45 minutes at room temperature. The analysis was conducted using eIF2α and ATP in a concentration equal to Km, enabling the identification of competitive and non-competitive inhibitors. The enzymatic reaction was then stopped by p-eIF2a (S51) - Eu3+ and Flag-d2 antibodies that were taken up in a homogenous HTRF Transcreener buffer (Cisbio) containing 60 mM EDTA and incubated for 60 min. (final concentration 6 µl). The utilised detection method is based on phosphospecific antibodies directed against eIF2α labelled with a fluorescent donor of europium cryptate (Eu3+) of prolonged stability and antiflag antibodies labelled with a HTRF acceptor fluorophore d2. FRET signal was measured by a Synergy HT plate reader (BioTek).

Data analysis: Km value for ATP was calculated based on the Michaelis-Menten equation. Km value for eIF2α was analysed by the Hill equation. Data were analysed using SigmaPlot (Systat Software, San Jose, CA) or GraphPad Prism (GraphPad software, La Jolla, CA).

### Example 3

### Analysis of PERK and eIF2α phosphorylation levels in mammalian cells in response to the presence of 3-acetyl-6methyl-2,4-heptanedione

To assess the biological activity of 3-acetyl-6methyl-2,4-heptanedione (compound 42233) in cells, 5 cell lines were selected so that they represent, in the best possible way, both tissues included in neurodegenerative processes and the activity of PERK/eIF2α pathway:
- SH-SY5Y line, morphologically regarded as neural, commonly used in studies on Alzheimer's disease and Parkinson's disease
- CATH.a, brain-derived mice neural cells
- DI TNC1, rat's brain-derived astrocytes
- NHA, human astrocytes
- 293T, human embryonic kidney cells
- 3T3T, mice fibroblasts

Initially, on studied 3T3T, SHY5Y, CATH.a and DI TNC1) cell lines, a cytotoxicity assay was carried out using 3-acetyl-6-methyl-2,4-heptanedione (compound 42233) for 2 h in the following concentrations: 0.4 µM, 0.8 µM, 1.6 µM, 3.125 µM, 6.25 µM, 12.5 µM, 25 µM, and 50 µM. Cells untreated with neither the inhibitor nor thapsigargin were used as a reference group and their viability was assayed as 100%. Statistical analysis did not show a significant decrease in viability of assayed cells even in the highest concentrations.
Then, the activity of compound 42233 was analysed in the following concentrations:
- 3 µM, 6 µM, 12 µM, 25 µM and 50 µM - for DI TNC1 and CATH lines;
- 10 µM and 50 µM - for 293T and 3T3T lines.
Choice of controls for the experiment:
- Experiment control - cells untreated with neither the inhibitor nor thapsigargin
- Known control inhibitor of known activity - thapsigargin in a concentration of 500 nM
- Control protein - β-actin

The PERK kinase inhibition study using cell lines in vitro (eIF2α) was as follows. Cells were preincubated for 1 hour with a tested PERK inhibitor in a number of concentrations of 3 µM - 50 µM and then, to activate the intracellular stress of endoplasmic reticulum, treated with thapsigargin in a concentration of 500 nM and incubated for 2 hours. In the experiments, the positive control constituted cells treated only with thapsigargin (500 nM) and the negative control constituted cells not treated with any of the compounds. To prepare analysed samples, cells were harvested and then lysed using an EBC buffer (50 mM Tris-HCl, 120 mM NaCl, 0.5% NP-40) supplemented by a cocktail of protease and phosphatase inhibitors. The homogenate was frozen and stored at -80°C until assays were made.

The measurement of general protein concentration in analysed samples was made using the Bradford method. To determine protein concentration in tested samples, a calibration curve (reference) was plotted using a number of dilutions of bovine serum albumin. Analysed samples were diluted with the EBC buffer and then mixed in 1:1 ratio with a denaturing buffer 2x Laemmli containing 2-mercaptoethanol. Then, samples were denatured at 95°C for 10 minutes. Samples having 80 µg of total protein were separated by electrophoresis in 10% polyacrylamide gel in denaturing conditions (SDS-PAGE) on a vertical electrophoresis apparatus. After the separation by electrophoresis, proteins were transferred to a methanol-activated PVDF membrane. After the transfer, the membrane with proteins was stained with a 0.1% solution of Ponceau-S to show protein fractions. The stain was removed by washing in triplicate in a TBST solution containing 0.05% Tween 20. To show all analysed proteins, the membrane was cut in half at appropriate heights. Then, membranes were blocked for 1 hour at room temperature in 5% solution of skimmed cow's milk prepared in TBST solution containing 0.05% of Tween 20. In the next step, membranes were incubated for 16 hours at 4°C with specific primary antibodies: p-eIF2α, eIF2α, β-actin and then for 1 hour with secondary antibodies conjugated with horseradish peroxidase. The detection of immunological complexes was conducted by using an ECL chemiluminescence set. Results were visualised on a blue light-sensitive x-ray film. The densitometric measure of lines was made by using Gene Tools software (Syngene, Cambridge, UK). The amount of protein was normalized for a signal obtained for β-actin, a reference protein.

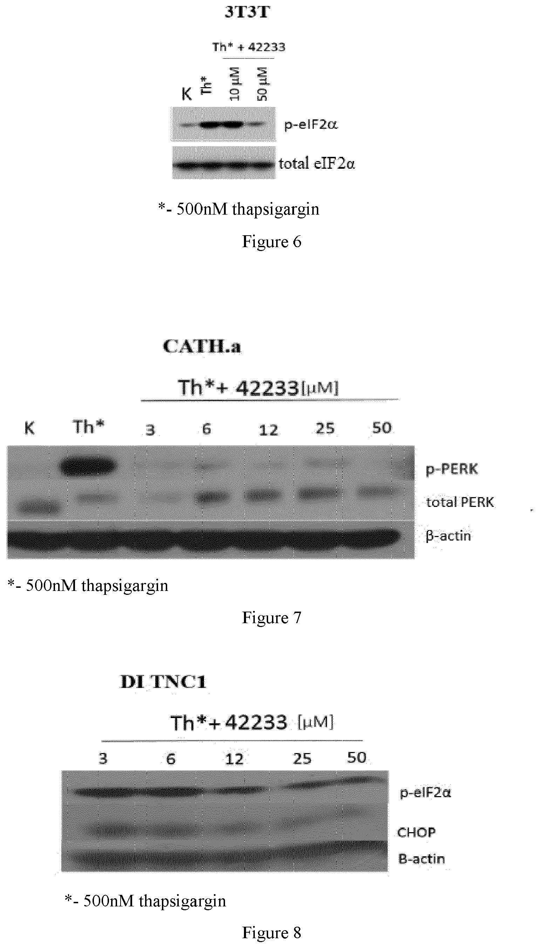

In the analysis of rat astrocytes, a 50% inhibition of eIF2α phosphorylation for concentrations 25 µM and 50 µM was shown (Figure 4). Similar results were obtained in murine fibroblasts and human embryonic kidney cells (Figure 5 and Figure 6). In the case of murine neural cells, full inhibition of PERK was shown for all used concentrations (Figure 7). Finally, a densitometric analysis of eIF2α phosphorylation level in response to 3-acetyl-6-methyl-2,4-heptanedione (compound 42233) for selected lines: murine neural cells CATH.a, rat astrocytes DI TNC1, and human astrocytes NHA showed a significant decrease of the phosphorylation level of this protein and therefore the inhibition efficacy of PERK kinase for analysed inhibitor concentrations from 3 µM to 50 µM, in a range of 15-49% relative to control cells treated only with thapsigargin.

### Example 4

### Influence of 3-acetyl-6-methyl-2,4-heptanedione (compound 42233) on CHOP level in neural cells

Apart from the global inhibition of protein synthesis through eIF2α phosphorylation, another effect of PERK-dependent response is the activation of ATF4 transcriptional factor that activates several genes, including a proaptotic CHOP. PERK inhibition can be therefore beneficial in two ways, additionally preventing the degeneration of neural tissue by apoptosis.

To assess the influence of 3-acetyl-6-methyl-2,4-heptanedione (designated as compound 42233) on CHOP level in neural cells, the following experiment was designed. Cells were preincubated for 1 hour with a tested PERK inhibitor in a number of concentrations of 3 µM - 50 µM and then, to activate the intracellular stress of endoplasmic reticulum, treated with thapsigargin in a concentration of 500 nM and incubated for 2 hours. In the experiments, the positive control included cells treated only with thapsigargin (500 nM) and the negative control included cells not treated with any of the compounds. To prepare analysed samples, cells were harvested and then lysed using an EBC buffer (50 mM Tris-HCl, 120 mM NaCl, 0.5% NP-40) supplemented by a cocktail of protease and phosphatase inhibitors. The homogenate was frozen and stored at -80°C until assays were made.

The measurement of general protein concentration in analysed samples was made using the Bradford method. To determine protein concentration in tested samples, a (reference) calibration curve was plotted using a number of dilutions of bovine serum albumin. Analysed samples were diluted with the EBC buffer and then mixed in 1:1 ratio with a denaturing buffer 2x Laemmli containing 2-mercaptoethanol. Then, samples were denatured at 95°C for 10 minutes. Samples having 80 µg of total protein were separated by electrophoresis in 10% polyacrylamide gel in denaturing conditions (SDS-PAGE) on a vertical electrophoresis apparatus. After the separation by electrophoresis, proteins were transferred to a methanol-activated PVDF membrane. After the transfer, the membrane with proteins was stained with a 0.1% solution of Ponceau-S to show protein fractions. The stain was removed by washing in triplicate in a TBST solution containing 0.05% Tween 20. To show all analysed proteins, the membrane was cut in half at appropriate heights. Then, membranes were blocked for 1 hour at room temperature in 5% solution of skimmed cow's milk prepared in TBST solution containing 0.05% Tween 20. In the next step, membranes were incubated for 16 hours at 4°C with specific primary antibodies: p-eIF2α, eIF2α, CHOP, β-actin and then for 1 hour with secondary antibodies conjugated with horseradish peroxidase. Detection of immunological complexes was conducted by using an ECL chemiluminescence set. Results were visualised on a blue light-sensitive x-ray film. The densitometric measure of lines was made by using Gene Tools software (Syngene, Cambridge, UK). The amount of protein was normalized for a signal obtained for β-actin, a reference protein.

It was shown that in rat astrocytes, 3-acetyl-6-methyl-2,4-heptanedione (designated in Figure 8 as compound 42233) caused a decrease of CHOP level of 15, 30, 45, 50 and 70% for concentrations of 3µM, 6 µM, 12 µM, 25 µM, 50 µM, consecutively.

### Example 5 Assessment of the undesired effect of 3-acetyl-6-methyl-2,4-heptanedione on mammalian cells

Another issue was to determine if the analysed inhibitor could have an undesirable side effect on a cell. To answer this question, a number of experiments were carried out to measure cytotoxicity, the course of the cell cycle and the possibility of inducing programmed cell death through apoptosis.

### a) Assessment of cell viability as affected by 3-acetyl-6-methyl-2,4-heptanedione - XTT analysis

First, a repeated cytotoxicity analysis of 3-acetyl-6-methyl-2,4-heptanedione (compound 42233) was made using a colorimetry test XTT based on a tetrazole salt reduction to formazan in prolonged inhibitor exposure conditions after 3, 16, 24, and 48 hours in the concentration range from 0.75 µM to extremely high 0.5 mM. The experiment was as follows: Cells were seeded on a 96-well cell culture plate in an appropriate culture medium in a concentration of 8x10⁶ cells. Then, cells were treated with the PERK inhibitor in a number of concentrations, 0.75 µM - 50 µM and 0.5 mM. Positive control constituted cells not treated with the analysed PERK inhibitor, whereas negative control constituted cells treated with 100% DMSO. To assess the cytotoxicity of solvent in the tested inhibitor, cells were incubated with 0.01% DMSO. Each analysed test was performed in triplicate. Cells were incubated for 3, 16, 24 and 48 hours. Then, to each test was added 25 µl of XTT/PMS mixture. Cells were incubated for 2 hours at 37°C, 5% CO₂ and 95% humidity. Absorbance was measured at 450 nm wavelength using Synergy HT plate reader (BioTek).

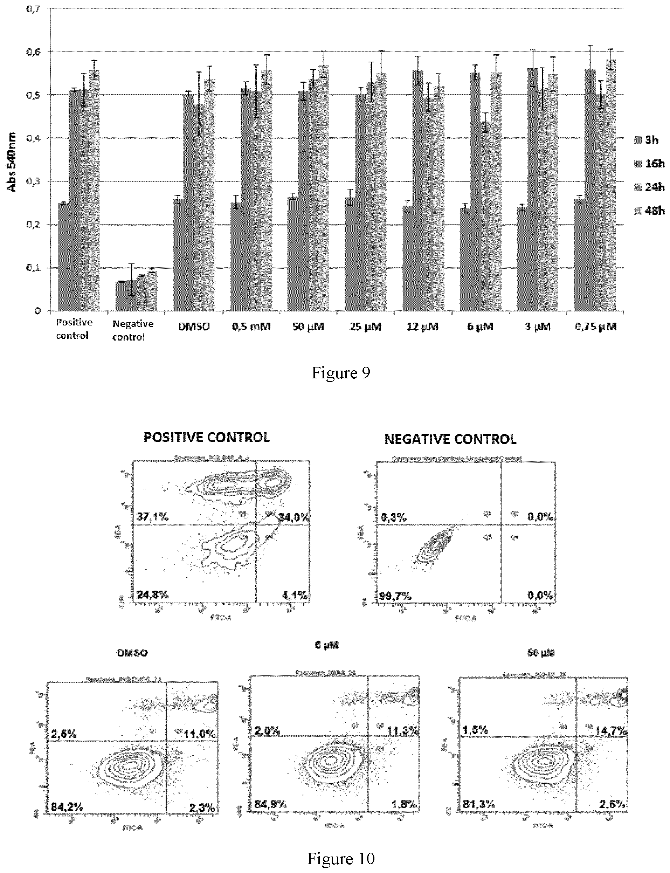

Obtained results did not show statistically significant decrease of viability (Figure 9) and therefore it should be noted that the analysed compound is not toxic for DI TNC1 line cells.

### b) Assessment of apoptosis induction by 3-acetyl-6-methyl-2,4-heptanedione

The analysis of apoptosis level in cells treated with the PERK inhibitor (i.e. compound 42233) was made using flow cytometry with double staining by Annexin V (Ann-V) and propidium iodide (PI).
Choice of controls for the experiment:
- Experiment control - cells treated with DMSO as a solvent for 3-acetyl-6-methyl-2,4-heptanedione (i.e. compound 42233)
- Staurosporine - as a known protein kinase inhibitor and known proapoptotic factor
Cells were preincubated for 1 hour with the PERK inhibitor (i.e. compound 42233) in concentrations of 6 µM and 50 µM, and then treated with thapsigargin as the endoplasmic reticulum stress activator in a concentration of 500 nM. Then, cells were incubated for 24 hours. Positive control constituted cells treated with 1 µM of staurosporine as an apoptosis inductor and then incubated for 16 hours, whereas negative control constituted cells not treated with any of the compounds and incubated for 24 hours. Additional control was cells incubated for 24 hours with 0.01% DMSO as a solvent for the tested PERK inhibitor. Then cells were harvested and washed in 1 X DPBS and then in a concentration of 1 X 10⁶/mL suspended in 1 X Binding Buffer. 1 X 10⁵ of cells were transferred to new Eppendorf vials, and then 5 µL were stained with Ann-V and 5 µL with PI. After incubating for 15 minutes in darkness at room temperature, samples were diluted with 400 µL 1 X Binding Buffer and analysed by cytometry using a FACS Canto II flow cytometer (Becton Dickinson).

In the studies, no influence of tested inhibitor (compound 42233) on the increase of apoptosis level in DI TNC1 line cells was observed (Figure 10) both for a low concentration 6 µM, as well as the upper limit used in 50 µM concentration inhibition tests, which showed activity comparable to the one of DMSO (solvent control). The tested compound has no necrotic activity on neural cells. Results are shown in Figure 10.

### c) Assessment of 3-acetyl-6-methyl-2,4-heptanedione (compound 42233) on cell cycle

In the last step, the influence of the tested PERK inhibitor on a cell cycle progression was analysed using flow cytometry with propidium iodide staining. In this embodiment, for analysis as reference concentrations of compound 42233, a low concentration of 6 pM and an upper limit used in inhibition tests, i.e. 50 pM, was used.
Choice of controls for the experiment:
- Test control are cells untreated with neither compound 42233 nor nocodazole
- Nocodazole - a control compound with a known effect on cell cycle

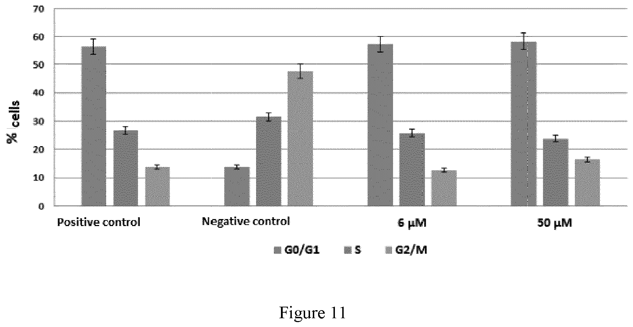

Cells in 10 cm culture dishes were treated with the PERK inhibitor in concentrations of 6 pM and 50 pM and then incubated for 24 hours. Positive control constituted cells treated with 1 pM nocodazole and incubated for 18 hours and negative control constituted cells not treated with any of the compounds. Cells were harvested and washed twice with 1 X DPBS and then fixed in 70% ethanol. Cells were stained with a mixture containing 50 pg/mL PI, 0.1 mg/mL RNase A and 0.6% NP-40 buffer. After 30 minutes of incubation at 4°C, tested samples were subject to cytometric analysis by using a FACS Canto II flow cytometer (Becton Dickinson). Obtained experimental data showed a lack of effect of 3-acetyl-6-methyl-2,4-heptanedione on the cell cycle in tested cells of DI TNC1 line (Figure 11).

## Claims

1. A compound of formula 1: 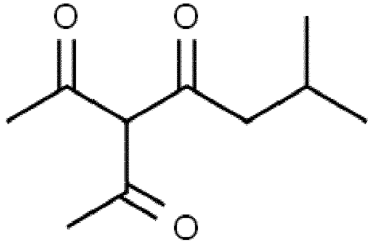 for use as the PERK kinase inhibitor.

2. The compound for use according to claim 1, **characterized in that** it is in a salt form.

3. The compound for use according to claim 1 or 2, **characterized in that** it is intended for use in preventing and/or treating diseases associated with endoplasmic reticulum stress.

4. The compound for use according to claim 3, **characterized in that** diseases associated with endoplasmic reticulum stress are selected from a group comprising glaucoma, Alzheimer's disease, Parkinson's disease.

5. The compound of formula 1: 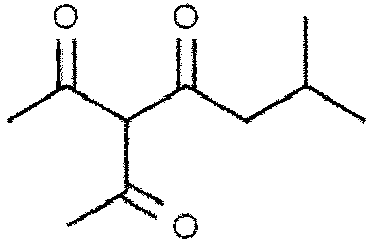 for use in preventing and/or treating diseases associated with endoplasmic reticulum stress.

6. The compound for use according to claim 5, **characterized in that** the compound of formula 1 is in a salt form.

7. The compound for use according to claim 5 or 6, **characterized in that** diseases associated with endoplasmic reticulum stress are selected from a group comprising glaucoma, Alzheimer's disease, Parkinson's disease.
